Europäisches Patentamt

European Patent Office

Office européen des brevets

(11) Publication number: **0 362 490 B1**

# EUROPEAN PATENT SPECIFICATION

(45) Date of publication of patent specification: **13.04.94**  (51) Int. Cl.5: **A61K 31/43**

(21) Application number: **89112354.9**

(22) Date of filing: **06.07.89**

The file contains technical information submitted after the application was filed and not included in this specification

(54) **A pharmaceutical composition having synergistic effect based on sulbactam and methampicillin.**

(30) Priority: **08.07.88 KR 850388**

(43) Date of publication of application:
**11.04.90 Bulletin  90/15**

(45) Publication of the grant of the patent:
**13.04.94 Bulletin  94/15**

(84) Designated Contracting States:
**DE ES FR GB IT**

(56) References cited:
**US-A- 4 234 579**
**US-A- 5 049 384**

**CHEMICAL ABSTRACT, vol. 20, 1987, Columbus, OH (US); D.BARBA et al., p. 6, no. 112473r&NUM;**

(73) Proprietor: **Kim, Yeong Sul**
**Cosmos Mansion n 1002**
**302-62 Ichon-Dong**
**Yongsan-ku Seoul(KR)**

(72) Inventor: **Kim, Yeong Sul**
**Cosmos Mansion n 1002**
**302-62 Ichon-Dong**
**Yongsan-ku Seoul(KR)**

(74) Representative: **Kraus, Walter, Dr. et al**
**Patentanwälte Kraus, Weisert & Partner**
**Thomas-Wimmer-Ring 15**
**D-80539 München (DE)**

EP 0 362 490 B1

**Description**

Sulbactam is a known $\beta$-lactam antibiotic, had its own antibacterial activity and strong affinity to the enzyme of $\beta$-lactamase, combines irreversiblly with the said enzyme and depresses the activity of the said enzyme. So, it is reported that when sulbactam is used as a combined form with other $\beta$-lactam antibiotics, the antibacterial activity is surprisingly increased [Fu, K.P, and Neu, H.C. Comparative inhivision of $\beta$-lactamase by novel $\beta$-lactam compounds, Antimicrob. Agents and Chemother. 15, 171-6(1979)].

Methampicillin is also a known $\beta$-lactam antibiotic and the antimicrobial activity is greatly decreased by $\beta$-lactamase. So, methampicillin is not used so much nowadays (British Patent No. 1,081,093).

In fact, it was reported that the activities of cephalosporins and penicillins were greatly increased when the antibiotics were combined with the small contents of clavulanic acid or sulbactam [Greenwood, D. and Eley, A.: In-Vitro evaluation of sulbactam, a penicillanic acid sulfone with $\beta$-lactam inhibitory properties, J. Antimicrob. Chemother. 10, 117-123(1982)].

An intensive study on the fields of combinations therefor was performed, However, no study on the combinations of sulbactam and methampicillin was carried out until now.

According to the invention, there are provided pharmaceutical composition having synergistic effect, useful for treating bacterial infections in mammals. Said composition comprises sulbactam or pharmaceutically acceptable salt thereof and methampicillin or pharmaceutically acceptable salt thereof.

This invention relates to a novel composition for medicinal use. More particularly, this invention relates to an anti-bacterial composition for medicinal use comprising sulbactam [penicillanic acid 1,1-dioxide] or pharmaceutically acceptable salt thereof and methampicillin or pharmaceutically acceptable salt thereof, which have an eminent synergistic effect against pathogens. The above mentioned pharmaceutically acceptable salts are those which are commonly used as salts of penicillanic acid derivatives with metals such as sodium, potassium and calcium, ammonium salt and salts with amines such as procain, dibenzylamine, N-benzyl-$\beta$-phenethylamine, 1-ethanamine and N,N-dibenzylethylenediamine. Therefore, the object of this invention is to provide an antimicrobial composition having synergistic effect against pathogenic bacteria which are resistant to conventional penicillins.

The suitable ratio of the sulbactam to methampicillin in the composition of this invention varies to some degrees depending on the types of target pathogenic bacteria or symptoms, but is generally in the range of from 1:10 to 10:1 (in the terms of potency ratio).

The antibacterial composition for medicinal use according to the present invention can be administered alone or it can be mixed with pharmaceutically acceptable carriers or diluents. They can be administered orally or parenterally.

The inventor isolated pathogens resistant to penicillins and studied the activity of $\beta$-lactamase produced by pathogenic bacteria resistant to penicillins would be changed by applying sulbactam alone (It refers to as "compound A"), methampicillin alone(It refers to as "compound I") and combination of sulbactam and methampicillin(It refers to as "combination A + I") or not.

1. Test Method :

1) Clinically isolated strains :

Strains were isolated from the patients infected with pneumonia hospitalized in Saint Mother Hospital and the isolated strains were passive-cultured and used in the test.

The strains were listed in the Table I. MIC test was performed by use of the strains listed in the Table II.

Table I

| Strains and numbers used in the test | |
|---|---|
| Strains | Numbers |
| S. aureus | 25 |
| E. coli | 8 |
| Ps. aeruginosa | 21 |
| Kl. pneumoniae | 9 |
| Ser. marcescens | 3 |
| S. epidermidis | 2 |
| Ent. cloacae | 9 |
| Pro. vulgaris | 1 |
| Pro. mirabilis | 1 |
| Actinobacter calcoaceticus | 1 |

Table II : MIC( µg/ml) of the compound I against the standard strains

| Antibiotics Strains | Compound I |
|---|---|
| S. aureus A. 6538P | 1 |
| E. coli A. 25922 | 4 |
| Kl. pneumoniae H7-9 | 100 |
| Ser. marcescens YH. S3 | 100 |
| S. epidermidis A. 12228 | 16 |
| Ent. cloacae H 30-4 | 2 |
| Pro. vulgaris A. 6059 | 1 |
| Pro. mirabilis A. 25933 | 1 |
| Ps. aeruginosa A. 27853 | 4 |

2. Antibiotic used :

Compound A and compound I were dissolved in distilled water, filtered and sterilized.

3. Activity Test According to Solid Culture Dilution Method (MIC Test) :

This test was carried out following the Correction Method of MIC Test Method suggested by Japanese Chemotherapy Society [Chemotherapy 29, 76-79(1981)].
That is,

1) MIC determination of compound I :

NB agar plates of containing 100, 8, 4, 2, 1 and 0.1 µg/ml of the test compounds I were prepared and the plates were divided corresponding to the test groups.
The test strains which were cultured for one night at 37°C were streaked on the plates of different

3

concentrations. The plates were cultured at 37°C for one night and the antibiotic concentration in which the growth of strains being not observed were made as the MIC.

2) MIC determination of compound A :

Agar plates of containing 128, 64, 32, 16, 8, 4, 2, 1, 0.1 μg/ml of compound A were prepared and the plates were divided corresponding to the test groups.

The selected strains from the above test 1) were inoculated in NB liquid cultures and cultured for one night at 37°C and were streaked on the agar plates prepared above. The plates were cultured at 37°C for one night and the antibiotic concentration in which the growth of the strains being not observed were made as the MIC.

4. Activity test of the combinations of compound A + I (synergistic activity) :

Agar plates of containing 100, 16, 8, 4, 2, 1, 0.1 μg/ml of the compound I,agar plates of simultaneously containing 16 μg/ml of compound A which corresponds to one-fourth of MIC of the compound A and containing the said 7 concentrations of the compound I each and agar plates of containing 16 μg/ml of the compound A alone were prepared and were divided corresponding to the test groups. The test strains cultured in NB culture for one night at 37°C were streaked on the plates prepared.

And the antibiotic activity of the compound I alone and the combinations of each compound I and compound A were determined and compared each other and the synergistic activity were determined.

5. Results :

1) MIC of compound I against standard strains :

The MIC of the compounds against the standard strains were determined and the results were shown in the Table II, Compound I showed susceptibility against the standard strains of S. aureus, E. coli, S. epidermidis, Ent. cloacae, Pro. vulgaris and Pro. mirabilis.

So, among the clinically isolated strains, the standard strains showed susceptibility. And whether the strains which showed resistant to the compound I among the clinically isolated strains showed susceptibility by combinations of compound A + I or not were tested.

2) MICs of compound A against the strains selected :

The MICs of compound A against 34 kinds of strains selected were determined and showed in the Table III, column of compound A. MICs of compound A against the 34 kinds of strains selected were distributed from 64 μg/ml to 128 μg/ml. That is, the MICs of 64 μg/ml were 10 kinds of strains, the MICs of 128 μg/ml, 9 kinds of strains and the MICs of more than 128 μg/ml, 15 kinds of strains.

3) The MICs of the combinations of compound I + compound A :

The concentration of compound A of 16 μg/ml which did not show susceptibility (One-fourth of the MIC of compound A) were fixed and the activity of compound I at this concentration of compound A and, when the compound I was simultaneously combined at the series of concentrations of dilutions of the compound I was determined. Table III showed the activity of the combinations of compound I and compound A.

Among total 34 kinds of strains, the MICs of 30 kinds of strains were decreased to the extent of from minimum 4 times to maximum 100 times or more of the MICs of the compound I. Especially, the MICs of each 7 kinds of strains among the 8 kinds of strains of each S. aureus and E. coli, were decreased.

4

Table III : Activity of the combinations of compound A +
compound I against comparatively resistant to
compound I (MIC : µg/ml)

| Strains \ Antibiotics | compound I | compound A | compound A+I |
|---|---|---|---|
| S. aureus A. 6538P | 1 | 128 | 1 |
| S. aureus A | 100 | >128 | 1 |
| B | 8 | >128 | 1 |
| C | 8 | >128 | 1 |
| D | 100 | >128 | 1 |
| E | > 100 | 128 | 1 |
| F | 8 | 128 | 1 |
| G | 100 | 128 | 2 |
| H | > 100 | >128 | 100 |
| E. coli A. 25922 | 4 | 64 | 1 |
| E. coli A | 100 | 64 | 8 |
| B | 100 | 128 | 100 |
| C | 8 | 64 | 1 |
| D | >100 | 64 | 1 |
| E | 8 | 64 | 1 |
| F | >100 | 64 | 2 |
| G | >100 | 64 | 2 |
| H | >100 | 64 | 2 |
| S. epidermidis A. 12228 | 16 | 64 | 1 |
| S. epidermidis A | >100 | >128 | 8 |
| B | 100 | >128 | 4 |
| Ent. cloacae H 30-4 | 2 | 64 | 1 |
| Ent. cloacae B | 100 | 128 | 4 |
| D | 100 | 128 | 1 |
| Ps. aeruginosa A 27853 | 4 | 128 | 4 |
| Ps. aeruginosa A | >100 | >128 | 8 |
| D | >100 | >128 | 4 |
| E | >100 | >128 | 16 |
| F | 16 | >128 | 4 |
| G | >100 | >128 | 100 |

Table III : (continued)

| Antibiotics / Strains | compund I | compound A | compound A+I |
|---|---|---|---|
| Ps. aeruginosa H | 16 | >128 | 4 |
| I | >100 | >128 | 4 |
| K | >100 | >128 | >100 |
| L | 100 | >128 | 16 |

* Strains are those isolated respectively from different patents.

The above data shows that the composition of the present invention shows a surprising synergistic effect against pathogenic bacteria resistant to penicillins.

Acute Toxicity :

10 Male mice of the ICR Strain of 4-5 meeks of age were administered orally the preparation of example 1 by a syringe and observed the mice 2 days after.
The $LD_{50}$ (mg/kg) was over 10,000.

| Example 1 (capsule) | |
|---|---|
| Methampicillin | 250.00mg |
| Sulbactam | 125.00mg |
| Magnesium stearate | 2.00mg |
| Lactose(qs) | 500.00mg |

The above composition was mixed and encapsulated with common method in pharmaceutical industry.

| Example 2 (Injection) | |
|---|---|
| Sodium methampicillin | 250.00mg |
| Sodium sulbactam | 250.00 |
| Distilled water for injection(qs) | 5 ml |

The above composition was dissolved in distilled water for injection and filled in an ampoule common method in pharmaceutical industry.

## Claims

**Claims for the following Contracting States : DE, GB, FR, IT**

1. A pharmaceutical composition having a synergistic effect comprising as active ingredients sulbactam or a pharmaceutically acceptable salt thereof and methampicillin or a pharmaceutically acceptable salt thereof.

2. The pharmaceutical composition for injection of claim 1, wherein the active ingredients are admixed with conventional carriers, diluents, stabilizing agents.

3. The pharmaceutical composition of claim 1, wherein the active ingredients are admixed in the ratio of from 1 : 9 to 9 : 1.

**Claims for the following Contracting State : ES**

1. A process for preparing a pharmaceutical composition having a synergistic effect comprising mixing as active ingredients sulbactam or a pharmaceutically acceptable salt thereof and methampicillin or a pharmaceutically acceptable salt thereof.

2. A process for preparing a pharmaceutical composition for injection according to claim 1, wherein the active ingredients are admixed with conventional carriers, diluents and stabilizing agents.

3. A process for preparing a pharmaceutical composition according to claim 1, wherein the active ingredients are admixed in the ratio of from 1 : 9 to 9 : 1.

**Patentansprüche**
**Patentansprüche für folgende Vertragsstaaten : DE, GB, FR, IT**

1. Pharmazeutische Zusammensetzung mit synergistischer Wirkung, dadurch **gekennzeichnet,** daß sie als aktive Bestandteile Sulbactam oder ein pharmazeutisch annehmbares Salz davon und Methampicillin oder ein pharmazeutisch annehmbares Salz davon enthält.

2. Pharmazeutische Zusammensetzung für die Injektion nach Anspruch 1, dadurch **gekennzeichnet,** daß die aktiven Bestandteile mit üblichen Trägern, Verdünnungsmitteln, Stabilisatoren vermischt sind.

3. Pharmazeutische Zusammensetzung nach Anspruch 1, dadurch **gekennzeichnet,** daß die aktiven Bestandteile in einem Verhältnis von 1:9 bis 9:1 vermischt sind.

**Patentansprüche für folgenden Vertragsstaat : ES**

1. Verfahren zur Herstellung einer pharmazeutischen Zusammensetzung mit synergistischer Wirkung, dadurch **gekennzeichnet,** daß als aktive Bestandteile Sulbactam oder ein pharmazeutisch annehmbares Salz davon und Methampicillin oder ein pharmazeutisch annehmbares Salz davon vermischt werden.

2. Verfahren zur Herstellung einer pharmazeutischen Zusammensetzung für die Injektion nach Anspruch 1, dadurch **gekennzeichnet,** daß die aktiven Bestandteile mit üblichen Trägern, Verdünnungsmitteln, Stabilisatoren vermischt sind.

3. Verfahren zur Herstellung einer pharmazeutischen Zusammensetzung nach Anspruch 1, dadurch **gekennzeichnet,** daß die aktiven Bestandteile in einem Verhältnis von 1:9 bis 9:1 vermischt sind.

**Revendications**
**Revendications pour les Etats contractants suivants : DE, GB, FR, IT**

1. Composition pharmaceutique ayant un effet synergique a, comprenant comme principes actifs, le sulbactam ou un sel a de celui-ci, acceptable du point de vue pharmaceutique, et la méthampicilline ou un sel a de celle-ci, acceptable du point de vue pharmaceutique.

2. Composition pharmaceutique suivant la revendication 1, dans laquelle les principes actifs sont mélangés avec les véhicules, diluants, et agents stabilisants conventionnels.

3. Composition pharmaceutique suivant la revendication 1, dans laquelle les principes actifs sont mélangés dans un rapport compris entre 1 : 9 et 9 : 1.

7

**Revendications pour l'Etat contractant suivant : ES**

1. Procédé de préparation d'une composition pharmaceutique ayant un effet synergique a, comprenant le mélange, en tant que principes actifs, de sulbactam, ou d'un sel a de celui-ci, acceptable du point de vue pharmaceutique, et de méthampicilline, ou d'un sel a de celle-ci, acceptable du point de vue pharmaceutique.

2. Procédé de préparation d'une composition pharmaceutique pour injection, suivant la revendication 1, dans laquelle les principes actifs sont mélangés avec les véhicules, diluants et agents stabilisants conventionnels.

3. Procédé de préparation d'une composition pharmaceutique suivant la revendication 1, dans laquelle les principes actifs sont mélangés dans un rapport compris entre 1 : 9 et 9 : 1.